# EUROPÄISCHE PATENTSCHRIFT

(11) **EP 2 614 849 B1**
(45) Veröffentlichungstag und Bekanntmachung des Hinweises auf die Patenterteilung: **21.06.2017**
(21) Anmeldenummer: 13158374.2
(22) Anmeldetag: 09.01.2007
(51) Int. Cl.: A61M 15/00

(54) **Dispergiereinheit**
Dispersion unit
Unité de dispersion

(30) Priorität: 17.02.2006 DE 102006007495
(43) Veröffentlichungstag der Anmeldung: 17.07.2013
(62) Teilanmeldung aus: 07702637.5
(73) Patentinhaber: Sanofi SA, 1214 Vernier (CH)
(72) Erfinder: Jauernig, Jürgen, 79539 Lörrach (DE); Weuthen, Thomas, 07407 Rudolstadt (DE); Mackeben, Stefan, 89073 Ulm (DE)
(74) Vertreter: Zwicker, Jörk

(56) Entgegenhaltungen:
- EP-A1- 1 068 874
- WO-A1-99/39761
- US-A- 5 954 047
- US-A1- 2004 107 963

## Beschreibung

Die vorliegende Erfindung betrifft eine Dispergiereinheit für einen Pulverinhalator. Derartige Dispergiereinheiten sind grundsätzlich bekannt und dienen dazu, eine Dispergierung eines Aerosols zu erzeugen, wobei das Aerosol aus einer Mischung aus Wirkstoff und Trägersubstanz, z.B. Lactose, besteht. Die Trägersubstanz dient hauptsächlich dazu, die physikalischen Eigenschaften der Formulierung, wie z.B. deren Fließfähigkeit zu kontrollieren. Dabei haftet der feine Wirkstoff vorwiegend auf der Oberfläche der groben Trägersubstanz. Die zwischen Träger- und Wirkstoffpartikeln, bzw. zwischen Wirkstoffpartikelagglomeraten bestehenden Adhäsionskräfte müssen während der Inhalation überwunden werden, um einen hohen Anteil lungengängiger Wirkstoffpartikel zu generieren. Die dazu notwendige Energie kann in einer Dispergiereinheit eingebracht werden. Bei bekannten Dispergiereinheiten werden Impaktionskräfte oder Turbulenzen oder eine Kombination aus beidem zur Dispergierung eingesetzt. Auch ist es bekannt, mit Hilfe von Prallwänden und zusätzlichen Zuluftkanälen eine Dispergierung zu erzeugen. Das Patentschrift US5954047A offenbart eine Dispergiereinheit für einen Pulverinhalator, in dem ein Ringkanal zum Zuführen eines Partikelstroms vorgesehen ist, der einen axialen Einlass und einen axialen Auslass aufweist, wobei sich an den axialen Auslass eine ringförmige Umlenkkammer anschließt, in welcher der axial eintretende Partikelstrom in eine überwiegend radiale Strömungsrichtung umgelenkt wird. An die Umlenkkammer ist in axialer Richtung eine Rotationskammer mit einer kreisförmigen Umfangswand und einem axialen Auslass angeschlossen. Der axiale Auslass der Rotationskammer ist zentrisch angeordnet und an den axialen Auslass der Rotationskammer schließt sich ein Austragskanal an.

Es ist die Aufgabe der Erfindung, eine Dispergiereinheit zu schaffen, die äußerst kompakt baut, konstruktiv einfach ist und mit der ohne Saugkraftverlust eine möglichst hohe Feinpartikelfraktion erzeugt werden kann.

Die Lösung dieser Aufgabe erfolgt durch die Merkmale des Anspruchs 1 und insbesondere durch eine Dispergiereinheit mit einem Mundstück, in dem ein Ringkanal zum Zuführen eines Partikelstroms vorgesehen ist. Hierbei weist der Ringkanal einen axialen Einlass und einen axialen Auslass auf, um den Partikelstrom, bestehend aus einer Mischung aus Wirkstoff und Trägersubstanz zuzuführen. Erfindungsgemäß schließt sich an den axialen Auslass des Ringkanals eine ringförmige Umlenkkammer an, in welcher der axial eintretende Partikelstrom in eine überwiegend radiale Strömungsrichtung umgelenkt wird. Gleichzeitig kann in dieser Umlenkkammer eine Beschleunigung des Partikelstroms erreicht werden, so dass der Partikelstrom in einer sich in axialer Richtung an die Umlenkkammer anschließenden Rotationskammer mit einer kreisförmigen Umfangswand und einem axialen Auslass kreisförmig umläuft.

Alleine durch Ansaugen an dem Mundstück kann also der durch den Ringkanal zugeführte Partikelstrom nach Austreten aus der Umlenkkammer in eine ringförmige Umlaufbahn gebracht werden, wobei leichte Partikel, beispielsweise reine Wirkstoffpartikel mit einer Teilchengröße weniger als 5 µm, aufgrund ihrer geringeren Zentrifugalkraft aus dem axialen Auslass der Rotationskammer frühzeitig austreten können. Andererseits werden gröbere Partikel, beispielsweise wirkstoffbeladene Trägerpartikel auf grund ihrer Massenträgheit länger in der Rotationskammer gehalten, in der sie mehrfach umlaufen und dabei gegen die Umfangswand der Rotationskammer prallen, wodurch sich zusätzlich die feinen Wirkstoffpartikel von dem gröberen Trägerpartikel lösen. Sämtliche feinen Partikel folgen dem Luftstrom durch den axialen Auslass der Rotationskammer mit verlangsamter Geschwindigkeit und stehen zur Inhalation als nicht ballistisches Aerosol bereit. Erfindungsgemäß werden die Umlenkkammer und die Rotationskammer nicht zum Abscheiden grober Partikel verwendet, sondern es wird eine sich in der durchschnittlichen Verweildauer unterscheidende Verteilung zwischen groben und feinen Partikeln ausgenutzt. Bis zum Ende des Inhalationsvorgangs können so auch gröbere Partikel die Rotationskammer verlassen, so dass keine nennenswerten Pulverrückstände verbleiben, welche die Funktionsfähigkeit des Inhalators bzw. die Gleichförmigkeit des Dosisaustrags bei Applikation weiterer Dosen verschlechtern könnten.

Der Ringkanal besitzt erfindungsgemäß einen axial orientierten Einlass und Auslass. Grundsätzlich kann jedoch der in den Ringkanal eingeführte Partikelstrom dennoch auch tangentiale Strömungskomponenten besitzen.

Vorteilhafte Ausführungsformen der Erfindung sind in der Beschreibung, der Zeichnung sowie den Unteransprüchen beschrieben.

Nach einer ersten vorteilhaften Ausführungsform können in der Umlenkkammer Leitschaufeln angeordnet sein, die schräg zur Axialrichtung orientiert sind. Mit derartigen Leitschaufeln lässt sich der axial über einen Ringraum eintretende Partikelstrom auf einfache Weise in eine Tangentialströmung umlenken, wobei gleichzeitig durch die Gestaltung der Umlenkschaufeln eine Beschleunigung des Partikelstroms in der Umlenkkammer bewirkt werden kann.

Es ist vorteilhaft, wenn die Leitschaufeln gekrümmt sind, um die gewünschte Umlenk- und Beschleunigungswirkung zu erzielen. Hierbei kann es vorteilhaft sein, wenn die Krümmung der Leitschaufeln in Axialrichtung abnimmt. Hierdurch kann die Leitschaufel nach Art einer Turbinenschaufel gestaltet werden, um eine möglichst gute Umlenkung und Beschleunigung zu erzielen. In diesem Zusammenhang kann es auch vorteilhaft sein, wenn die Leitschaufeln im Schnitt das Profil eines Tragflügels mit gekrümmter Skelettlinie aufweisen. Auch kann es in diesem Zusammenhang vorteilhaft sein, wenn die Leitschaufeln im Bereich des Einlasses der Umlenkkammer eine gerundete Vorderkante und im Bereich des Auslasses der Umlenkkammer eine weniger stark gerundete Hinterkante aufweisen. Durchgeführte Testversuche haben gezeigt, dass durch eine solche Profilgestaltung sehr gute Ergebnisse erzielt werden können.

Nach einer weiteren vorteilhaften Ausführungsform ist der axiale Auslass der Rotationskammer zentrisch angeordnet. Hierdurch können leichte Partikel die Rotationskammer durch den Auslass frühzeitig verlassen, wohingegen schwerere Partikel entlang der Umfangswand der Rotationskammer umlaufen.

An den axialen Auslass der Rotationskammer schließt sich ein Austragskanal an, der sich erweitert. Die Erweiterung ist konkav ausgebildet. Dadurch kann erreicht werden , dass die Aerosolpartikel, die aus dem Auslass der Rotationskammer mit relativ hohen Geschwindigkeitskomponenten quer zur Inhalationsrichtung austreten, im Bereich des Austragskanals verlangsamt werden, wobei die Bewegung des Aerosols im Auslasskanal überwiegend in Längsrichtung orientiert wird. Gleichzeitig wird durch die Querschnittserhöhung des Austragskanals ein langsamer Aerosolaustritt erreicht, so dass der Patient ein nicht ballistisches Aerosol inhaliert. Mit einer solchen Mundstückgeometrie wird durch Beeinflussung der Austrittsrichtung sowie der Austrittsgeschwindigkeit die Aerosoldeposition im oropharygealen Bereich des Patienten reduziert. Obwohl das Aerosol aus der Rotationskammer in den Auslass mit relativ hohen Radialgeschwindigkeiten austritt, ist die Aerosolaustrittsgeschwindigkeit am Ende des Austragskanals relativ gering.

Weiter kann es vorteilhaft sein, wenn der Austragskanal in einem ausgangseitigen Endabschnitt einen kreiszylindrischen Bereich aufweist, da hierdurch eine axiale Bündelung des ausgetragenen Partikelstroms bewirkt werden kann. Statt einer konkaven Gestaltung des Austragskanals ist auch eine konvexe Ausbildung denkbar.

Die Abscheidung von leichten Partikeln aus der Rotationskammer kann zusätzlich dadurch verbessert werden, dass der Austragskanal scharfkantig, und insbesondere mit einer im Querschnitt spitzwinkligen Kante an die Rotationskammer anschließt.

Auch hat es sich als vorteilhaft erwiesen, in der Rotationskammer den Übergang von der kreisförmigen Umfangswand zu dem axialen Auslass teilweise gekrümmt auszubilden, da dies einerseits eine verbesserte Aerodynamik und andererseits ein vermindertes Ablagern von Partikeln bewirkt.

Bei der erfindungsgemäßen Dispergiereinheit sind zwischen dem axialen Auslass des Ringkanals und dem Auslass der Rotationskammer keinerlei Lufteintrittsöffnungen zum Zuführen von externer Luft vorgesehen. Hierdurch ist ausgeschlossen, dass eine zusätzliche Saugleistung zur Aufrechterhaltung der Funktionalität der Dispergiereinheit aufgebracht werden muss, die weder der Mobilisierung des Pulvers aus der Dispergiervorrichtung, noch der eigentlichen Dispergierleistung zugute kommt. Die Umlenkung des Partikelstroms und der gerichtete Auslass in den Rachenraum sind erfindungsgemäß allein über geometrische Ausformungen realisiert.

Nachfolgend wird die vorliegende Erfindung rein beispielhaft anhand einer vorteilhaften Ausführungsform und unter Bezugnahme auf die beigefügte Zeichnung beschrieben.

Es zeigt:

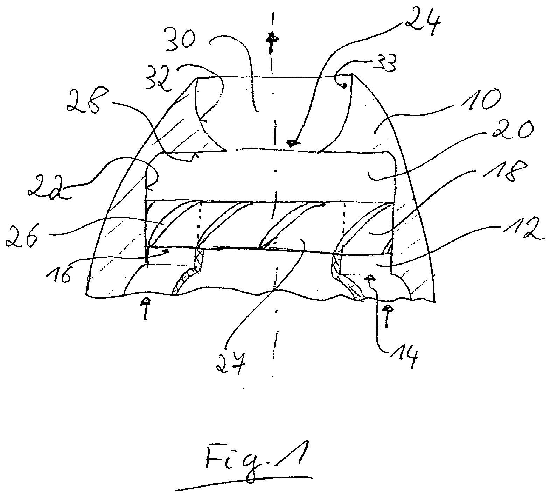

- Fig. 1: eine teilweise geschnittene Seitenansicht einer Dispergiereinheit.

Fig. 1 zeigt eine Dispergiereinheit für einen (nicht dargestellten) Pulverinhalator mit einem Mundstück 10, an dessen Unterseite ein Ringkanal 12 zum Zuführen eines Partikelstroms vorgesehen ist. Der Partikelstrom wird grundsätzlich durch Saugen an dem Mundstück erzeugt, beispielsweise indem in dem Inhalator eine vorbestimmte Dosis aus Wirkstoff und Trägersubstanz bereitgestellt wird, die dann durch Saugen an dem Mundstück in den Ringkanal 12 eingesaugt wird.

Der Ringkanal 12 ist in Umfangsrichtung umlaufend ausgebildet und weist einen axialen Einlass 14 und einen axialen Auslass 16 auf, wobei sich sowohl Einlass 14 als auch Auslass 16 über den gesamten Umfang des Ringkanals 12 erstrecken.

Im Anschluss an den axialen Auslass 16 des Ringkanals 12 ist eine ebenfalls ringförmige Umlenkkammer 18 vorgesehen, welche etwa die gleiche radiale Erstreckung wie der Ringkanal 12 besitzt und in welcher der axial eintretende Partikelstrom in eine überwiegend radiale Strömungsrichtung umgelenkt wird. Hierbei wird der im Wesentlichen radial gerichtete Partikelstrom am Auslass der Umlenkkammer 18 in eine Rotationskammer 20 geleitet, die eine kreisförmige Umfangswand 22 und einen axialen Auslass 24 aufweist.

Wie Fig. 1 zeigt, sind der Außendurchmesser des Ringkanals 12, der Umlenkkammer 18 sowie der Rotationskammer 20 im Wesentlichen gleich groß. Auch entsprechen der Innendurchmesser des Ringkanals 12 und der Innendurchmesser der Umlenkkammer 18 einander. Der Innendurchmesser des axialen Auslasses 24 der Rotationskammer 20 ist geringer als der Innendurchmesser der Umlenkkammer 18.

Um den axial eintretenden Partikelstrom in der Umlenkkammer 18 in eine überwiegend radiale Strömungsrichtung umzulenken und gleichzeitig zu beschleunigen, sind in der Umlenkkammer 18 über deren Umfang verteilt mehrere Leitschaufeln 26 vorgesehen, die schräg zur Axialrichtung orientiert sind. Jede der Leitschaufeln 26 erstreckt sich dabei über den gesamten Querschnitt der Umlenkkammer 18, wobei jede Leitschaufel gekrümmt ausgebildet ist und die Krümmung in Axialrichtung abnimmt, d.h. am Einlass der Umlenkkammer 18 stärker als am Auslass ist. Im Schnitt (Längsschnitt) weisen die Leitschaufeln 26 das Profil eines Tragflügels mit gekrümmter Skelettlinie auf. Nach einer vorteilhaften Ausführungsform weisen die Leitschaufeln im Bereich des Einlasses der Umlenkkammer 18 eine gerundete Vorderkante und im Bereich des Auslasses der Umlenkkammer 18 eine weniger stark gerundete Hinterkante auf, so dass das Profil der Leitschaufeln 26 einem Flugzeugflügel ähnelt.

Wie Fig. 1 ferner zeigt, ist die Umfangswand 22 der Rotationskammer 20 kreiszylindrisch ausgebildet und schließt sich unmittelbar an den Auslass der Umlenkkammer 18 an, wobei die axiale Erstreckung der Umlenkkammer 18 und der Rotationskammer 20 etwa gleich groß ist. An ihrem auslassseitigen Ende weist die Rotationskammer 20 eine Stirnwand 28 auf, welche einen Übergang zwischen der Umfangswand 22 und dem zentrisch angeordneten axialen Auslass 24 bildet. Hierbei ist der Übergang von der kreisförmigen Umfangswand 22 zu der Stirnwand 28 im Bereich der Ecke gekrümmt ausgebildet.

An den axialen Auslass 24 der Rotationskammer 20 schließt sich ein Austragskanal 30 an, dessen Umfangswand 32 sich konkav erweitert. Der Übergang zwischen der Stirnwand 28 der Rotationskammer 20 und der Umfangswand 32 des Austragskanals 30 ist jedoch scharfkantig und beim dargestellten Ausführungsbeispiel spitzwinklig ausgebildet. Ferner weist der Austragskanal 30 in seinem ausgangseitigen Endabschnitt einen kreiszylindrischen Bereich 33 auf, der der sich bis zum Ende des Austragskanals 30 erstreckt und der eine axiale Bündelung des ausgetragenen Partikelstroms bewirkt.

Wie die Fig. 1 ferner zeigt, sind zwischen dem Einlass 14 des Ringkanals 12 und dem Austragskanal 30 keine Lufteintrittsöffnungen zum Zuführen von externer Luft vorgesehen.

Beim Gebrauch der beschriebenen Dispergiereinheit saugt der Patient an dem Mundstück 10, wodurch ein Partikelstrom in Richtung der dargestellten Pfeile (Axialrichtung) durch das Mundstück hindurchgeführt wird, der von einem nicht dargestellten Pulverinhalator zuvor in einer gewünschten Dosis bereitgestellt worden ist. Der angesaugte Partikelstrom wird zunächst durch den Einlass 14 in den Ringkanal 12 eingeführt und tritt durch den ringförmigen, axialen Auslass 16 aus dem Ringkanal 12 in die ringförmige Umlenkkammer 18. In der Umlenkkammer 18 wird der Partikelstrom durch die Leitschaufeln 26 einerseits beschleunigt und andererseits in eine überwiegend radiale Strömungsrichtung umgelenkt, so dass der Partikelstrom am Auslass der Umlenkkammer 18 annähernd tangential in die Rotationskammer 20 eintritt, die sich in axialer Richtung an die Umlenkkammer 18 anschließt. In der Rotationskammer 20 rotiert der Partikelstrom, wobei schwere Partikel im Bereich der kreisförmigen Umfangswand 22 länger umlaufen und leichtere Partikel dem Luftstrom folgen und sich schneller in Richtung Austragskanal 30 bewegen.

Die in der Rotationskammer 20 umlaufenden schwereren Partikel geben zunächst während ihres Umlaufs durch Kontakt mit der Umfangswand 22 zunehmend kleinere (Wirkstoff-)Partikel ab, bis diese in der Rotationskammer 20 umlaufenden Partikel ebenfalls dem Luftstrom folgen und dann auch ausgetragen werden.

Die beschriebene Dispergiereinheit wird nach einer vorteilhaften Ausführungsform aus Kunststoff hergestellt. Hierbei kann es vorteilhaft sein, die Leitschaufeln 26 einstückig mit einem Einsatz 27 auszubilden, beispielsweise als Spritzgussteil, wobei der Einsatz 27 mit den daran angeformten Leitschaufeln 26 in das Innere des Mundstücks 10 eingesetzt werden kann.

Im folgenden Text werden Beispiele angegeben, wobei die einzelnen Beispiele nummeriert sind, um die Bezugnahme auf Merkmale einzelner Beispiele in anderen Beispielen zu erleichtern.
1. Dispergiereinheit für einen Pulverinhalator, mit einem Mundstück (10), in dem ein Ringkanal (12) zum Zuführen eines Partikelstroms vorgesehen ist, der einen axialen Einlass (14) und einen axialen Auslass (16) aufweist, wobei sich an den axialen Auslass (16) eine ringförmige Umlenkkammer (18) anschließt, in welcher der axial eintretende Partikelstrom in eine überwiegend radiale Strömungsrichtung umgelenkt wird, und wobei sich an die Umlenkkammer (18) in axialer Richtung eine Rotationskammer (20) mit einer kreisförmigen Umfangswand (22) und einem axialen Auslass (24) anschließt.
2. Dispergiereinheit nach Beispiel 1, wobei in der Umlenkkammer (18) schräg zur Axialrichtung orientierte Leitschaufeln (26) angeordnet sind.
3. Dispergiereinheit nach Beispiel 2, wobei die Leitschaufeln (26) eine Beschleunigung des Partikelstromes bewirken.
4. Dispergiereinheit nach Beispiel 2 oder 3, wobei die Leitschaufeln (26) gekrümmt sind, wobei die Krümmung in Axialrichtung abnimmt.
5. Dispergiereinheit nach Beispiel 2, 3 oder 4, wobei die Leitschaufeln (26) im Schnitt das Profil eines Tragflügels mit gekrümmter Skelettlinie aufweisen.
6. Dispergiereinheit nach einem der Beispiele 2 - 5, wobei die Leitschaufeln (26) im Bereich des Einlasses der Umlenkkammer (18) eine gerundete Vorderkante und im Bereich des Auslasses der Umlenkkammer (18) eine weniger stark gerundete Hinterkante aufweisen.
7. Dispergiereinheit nach zumindest einem der vorstehenden Beispiele, wobei der axiale Auslass (24) der Rotationskammer (20) zentrisch angeordnet ist.
8. Dispergiereinheit nach zumindest einem der vorstehenden Beispiele, wobei sich an den axialen Auslass (24) der Rotationskammer (20) ein sich insbesondere konkav erweiternder Austragskanal (30) anschließt.
9. Dispergiereinheit nach Beispiel 8 wobei der Austragskanal (30) scharfkantig, insbesondere unter einem im Querschnitt spitzen Winkel, an die Rotationskammer (20) anschließt.
10. Dispergiereinheit nach Beispiel 8 oder 9, wobei der Austragskanal (30) in einem ausgangseitigen Endabschnitt einen kreiszylindrischen Bereich (33) aufweist, um eine axiale Bündelung des ausgetragenen Partikelstroms zu bewirken.
11. Dispergiereinheit nach zumindest einem der vorstehenden Beispiele, wobei in der Rotationskammer (20) der Übergang (28) von der kreisförmigen Umfangswand (22) zu dem axialen Auslass (24) teilweise gekrümmt ist.
12. Dispergiereinheit nach zumindest einem der vorstehenden Beispiele, wobei der Auslass (16) des Ringkanals (12) sich über den gesamten Umfang erstreckt.
13. Dispergiereinheit nach zumindest einem der vorstehenden Beispiele, wobei zwischen dem axialen Auslass (16) des Ringkanals (12) und dem Auslass (24) der Rotationskammer (18) keine Lufteintrittsöffnungen zum Zuführen von externer Luft vorgesehen sind.

### Bezugszeichenliste

- 10: Mundstück
- 12: Ringkanal
- 14: Einlass des Ringkanals
- 16: Auslass des Ringkanals
- 18: Umlenkkammer
- 20: Rotationskammer
- 22: kreisförmige Umfangswand
- 24: Auslass der Rotationskammer
- 26: Leitschaufeln
- 27: Einsatz
- 28: Stirnwand
- 30: Austragskanal
- 32: Umfangwand
- 33: kreiszylindrischer Bereich

## Patentansprüche

1. Dispergiereinheit für einen Pulverinhalator, mit einem Mundstück (10), in dem ein Ringkanal (12) zum Zuführen eines Partikelstroms vorgesehen ist, der einen axialen Einlass (14) und einen axialen Auslass (16) aufweist, wobei sich an den axialen Auslass (16) eine ringförmige Umlenkkammer (18) anschließt, in welcher der axial eintretende Partikelstrom in eine überwiegend radiale Strömungsrichtung umgelenkt wird, und wobei sich an die Umlenkkammer (18) in axialer Richtung eine Rotationskammer (20) mit einer kreisförmigen Umfangswand (22) und einem axialen Auslass (24) anschließt, **dadurch gekennzeichnet, dass** der axiale Auslass (24) der Rotationskammer (20) zentrisch angeordnet ist und dass sich an den axialen Auslass (24) der Rotationskammer (20) ein sich konkav erweiternder Austragskanal (30) anschließt.

2. Dispergiereinheit nach Anspruch 1, **dadurch gekennzeichnet, dass** in der Umlenkkammer (18) schräg zur Axialrichtung orientierte Leitschaufeln (26) angeordnet sind.

3. Dispergiereinheit nach Anspruch 2, **dadurch gekennzeichnet, dass** die Leitschaufeln (26) eine Beschleunigung des Partikelstromes bewirken.

4. Dispergiereinheit nach Anspruch 2 oder 3, **dadurch gekennzeichnet, dass** die Leitschaufeln (26) gekrümmt sind, wobei die Krümmung in Axialrichtung abnimmt.

5. Dispergiereinheit nach Anspruch 2, 3 oder 4, **dadurch gekennzeichnet, dass** die Leitschaufeln (26) im Schnitt das Profil eines Tragflügels mit gekrümmter Skelettlinie aufweisen.

6. Dispergiereinheit nach einem der Ansprüche 2 - 5, **dadurch gekennzeichnet, dass** die Leitschaufeln (26) im Bereich des Einlasses der Umlenkkammer (18) eine gerundete Vorderkante und im Bereich des Auslasses der
Umlenkkammer (18) eine weniger stark gerundete Hinterkante aufweisen.

7. Dispergiereinheit nach zumindest einem der vorstehenden Ansprüche, **dadurch gekennzeichnet, dass** der Austragskanal (30) scharfkantig, insbesondere unter einem im Querschnitt spitzen Winkel, an die Rotationskammer (20) anschließt.

8. Dispergiereinheit nach zumindest einem der vorstehenden Ansprüche, **dadurch gekennzeichnet, dass** der Austragskanal (30) in einem ausgangseitigen Endabschnitt einen kreiszylindrischen Bereich (33) aufweist, um eine axiale Bündelung des ausgetragenen Partikelstroms zu bewirken.

9. Dispergiereinheit nach zumindest einem der vorstehenden Ansprüche, **dadurch gekennzeichnet, dass** in der Rotationskammer (20) der Übergang (28) von der kreisförmigen Umfangswand (22) zu dem axialen Auslass (24) teilweise gekrümmt ist.

10. Dispergiereinheit nach zumindest einem der vorstehenden Ansprüche, **dadurch gekennzeichnet, dass** der Auslass (16) des Ringkanals (12) sich über den gesamten Umfang erstreckt.

11. Dispergiereinheit nach zumindest einem der vorstehenden Ansprüche, **dadurch gekennzeichnet, dass** zwischen dem axialen Auslass (16) des Ringkanals (12) und dem Auslass (24) der Rotationskammer (20) keine Lufteintrittsöffnungen zum Zuführen von externer Luft vorgesehen sind.

## Claims

1. Dispersing unit for a powder inhaler, with a mouthpiece (10) in which an annular channel (12) which has an axial inlet (14) and an axial outlet (16) is provided for the supply of a particle flow, wherein the axial outlet (16) is adjoined by an annular deflection chamber (18) in which the axially incoming particle flow is deflected into a predominantly radial direction of flow, and wherein the deflection chamber (18) is adjoined in the axial direction by a rotation chamber (20) with a circular peripheral wall (22) and with an axial outlet (24), **characterized in that** the axial outlet (24) of the rotation chamber (20) is arranged centrally and **in that** the axial outlet (24) of the rotation chamber (20) is adjoined by a discharge channel (30) widening concavely.

2. Dispersing unit according to Claim 1, **characterized in that** guide vanes (26) oriented obliquely with respect to the axial direction are arranged in the deflection chamber (18).

3. Dispersing unit according to Claim 2, **characterized in that** the guide vanes (26) cause an acceleration of the particle flow.

4. Dispersing unit according to Claim 2 or 3, **characterized in that** the guide vanes (26) are curved, wherein the curvature decreases in the axial direction.

5. Dispersing unit according to Claim 2, 3 or 4, **characterized in that** the guide vanes (26) have, in cross section, the profile of an aerofoil with a curved mean line.

6. Dispersing unit according to one of Claims 2-5, **characterized in that** the guide vanes (26) have a rounded leading edge in the area of the inlet of the deflection chamber (18) and a less rounded trailing edge in the area of the outlet of the deflection chamber (18).

7. Dispersing unit according to at least one of the preceding claims, **characterized in that** the discharge channel (30) adjoins the rotation chamber (20) with a sharp edge, in particular at an angle acute in cross section.

8. Dispersing unit according to at least one of the preceding claims, **characterized in that** the discharge channel (30) has a circular cylindrical area (33) in an end portion at the outlet side, in order to effect an axial bundling of the discharged particle flow.

9. Dispersing unit according to at least one of the preceding claims, **characterized in that**, in the rotation chamber (20), the transition (28) from the circular peripheral wall (22) to the axial outlet (24) is partially curved.

10. Dispersing unit according to at least one of the preceding claims, **characterized in that** the outlet (16) of the annular channel (12) extends over the entire circumference.

11. Dispersing unit according to at least one of the preceding claims, **characterized in that** no air inlet openings for the supply of external air are provided between the axial outlet (16) of the annular channel (12) and the outlet (24) of the rotation chamber (20).

## Revendications

1. Unité de dispersion pour un inhalateur de poudre, comprenant un embout (10) dans lequel est prévu un canal annulaire (12) pour l'introduction d'un courant de particules, qui présente une entrée axiale (14) et une sortie axiale (16), une chambre de déviation (18) de forme annulaire se raccordant à la sortie axiale (16), le courant de particules entrant axialement étant dévié dans ladite chambre de déviation dans une direction d'écoulement essentiellement radiale, et une chambre de rotation (20) avec une paroi périphérique circulaire (22) et une sortie axiale (24) se raccordant à la chambre de déviation (18) dans la direction axiale, **caractérisée en ce que** la sortie axiale (24) de la chambre de rotation (20) est disposée centralement **en ce qu'**un canal de décharge (30) s'élargissant sous forme concave se raccorde à la sortie axiale (24) de la chambre de rotation (20).

2. Unité de dispersion selon la revendication 1, **caractérisée en ce que** des pales directrices (26) orientées obliquement par rapport à la direction axiale sont disposées dans la chambre de déviation (18).

3. Unité de dispersion selon la revendication 2, **caractérisée en ce que** les pales directrices (26) provoquent une accélération du courant de particules.

4. Unité de dispersion selon la revendication 2 ou 3, **caractérisée en ce que** les pales directrices (26) sont courbes, la courbure diminuant dans la direction axiale.

5. Unité de dispersion selon la revendication 2, 3 ou 4, **caractérisée en ce que** les pales directrices (26) présentent en coupe le profil d'une ailette porteuse ayant une ligne moyenne courbe.

6. Unité de dispersion selon l'une quelconque des revendications 2-5, **caractérisée en ce que** les pales directrices (26) présentent dans la région de l'entrée de la chambre de déviation (18) une arête avant arrondie et dans la région de la sortie de la chambre de déviation (18) une arête arrière moins fortement arrondie.

7. Unité de dispersion selon au moins l'une quelconque des revendications précédentes, **caractérisée en ce que** le canal de décharge (30) se raccorde à la chambre de rotation (20) par une arête vive, en particulier suivant un angle aigu en section transversale.

8. Unité de dispersion selon au moins l'une quelconque des revendications précédentes, **caractérisée en ce que** le canal de décharge (30) présente, dans une portion d'extrémité du côté de la sortie, une région cylindrique circulaire (33) afin de provoquer une mise en faisceau axiale du courant de particules déchargé.

9. Unité de dispersion selon au moins l'une quelconque des revendications précédentes, **caractérisée en ce que** dans la chambre de rotation (20), la transition (28) de la paroi périphérique circulaire (22) à la sortie axiale (24) est partiellement courbe.

10. Unité de dispersion selon au moins l'une quelconque des revendications précédentes, **caractérisée en ce que** la sortie (16) du canal annulaire (12) s'étend sur toute la périphérie.

11. Unité de dispersion selon au moins l'une quelconque des revendications précédentes, **caractérisée en ce qu'**entre la sortie axiale (16) du canal annulaire (12) et la sortie (24) de la chambre de rotation (20) ne sont prévues aucunes ouvertures d'entrée d'air pour l'alimentation en air extérieur.
